# EUROPEAN PATENT APPLICATION

(11) **EP 1 849 501 A2**
(43) Date of publication of application: **31.10.2007**
(21) Application number: 07008500.6
(22) Date of filing: 26.04.2007
(51) Int. Cl.: A61Q 19/00, A61K 8/46

(54) **Methods for peeling skin**

(30) Priority: 27.04.2006 US 411821
(71) Applicant: L'Oréal, 75008 Paris (FR)
(72) Inventor: Hansenne, Isabelle, Westfield New Jersey 07090 (US); Cornell, Marc, Jackson New Jersey 08527 (US)
(74) Representative: Le Coupanec, Pascale A.M.P.

(57) **Abstract**

The invention relates to methods for peeling skin comprising applying a pre-peeling composition comprising urea and an N-substituted aminosulfonic acid compound to the skin and subsequently applying a peeling composition to the skin, as well as to compositions and kits containing such compositions which are useful in such methods.

## Description

The present invention generally relates to methods for peeling skin comprising applying a pre-peeling composition comprising urea and an N-substituted aminosulfonic acid compound to the skin and subsequently applying a peeling composition to the skin, as well as to compositions and kits containing such compositions which are useful in such methods.

Skin peeling methods are generally known for improving the surface appearance of skin, in particular for attenuating pigmentation defects such as actinic lentigo or acne or varicella marks, and for smoothing irregularities of skin texture, in particular wrinkles and fine lines, by causing a targeted exfoliation of the epidermis and/or of the top layers of the dermis.

Conventional skin peeling procedures include mechanical skin removal, e.g., dermabrasion or CO₂ laser, and chemical-induced skin removal. Chemical skin peeling techniques are currently very popular and are often categorized by the degree or amount of skin removal effected.

Chemical peels may be categorized as superficial, medium and deep chemical peels, depending on the depth of chemical wounding of the skin that occurs. Superficial chemical peels are those which remove or effect accelerated replacement or replenishment of the epidermis. Medium depth peels penetrate to the papillary dermis. Deep peels penetrate to the reticular dermis. WO 97/28786 reports that chemical peeling agents include alpha-hydroxy acids ("AHA's"), e.g., glycolic acid or other "fruit acids" such as citric and lactic acids; trichloroacetic acid, phenol, resorcinol and Jessner's solution which is an ethanol solution containing resorcinol, salicylic acid and lactic acid.

However, a need remains for chemical peeling methods and composition which provide good results, preferably without (or with less) adverse side effects or drawbacks typically found with conventional chemical peels, agents and compositions. An illustrative adverse side effect of chemical peels is a burning sensation on/in skin which has been subjected to a chemical peel.

Accordingly, aspects of the present invention include peeling and pre-peeling compositions which, when used in peeling procedures, are able to provide good peeling results without (or with less) adverse side effects or drawbacks.

The present invention relates to methods, in particular cosmetic methods, for peeling skin comprising applying a pre-peeling composition comprising urea and an N-substituted aminosulfonic acid compound to the skin and subsequently applying a peeling composition to the skin.

The present invention also relates to methods, in particular cosmetic methods, for preparing skin for peeling comprising applying a pre-peeling composition comprising urea and an N-substituted aminosulfonic acid compound to the skin prior to applying a peeling composition to the skin.

The present invention also relates to methods, in particular cosmetic methods, for improving the tactile roughness of skin which has been peeled comprising applying a pre-peeling composition comprising urea and an N-substituted aminosulfonic acid compound to the skin prior to applying a peeling composition to the skin.

The present invention also relates to methods, in particular cosmetic methods, for minimizing the burning sensation on or in skin which has been peeled comprising applying a pre-peeling composition comprising urea and an N-substituted aminosulfonic acid compound to the skin prior to applying a peeling composition to the skin.

The present invention further relates to two compositions useful for peeling skin. The first composition is a pre-peeling composition comprising urea and an N-substituted aminosulfonic acid compound. The second composition is a peeling composition comprising a peeling agent.

The present invention also relates to kits useful for peeling skin. The kits comprise a pre-peeling composition comprising urea and an N-substituted aminosulfonic acid compound, and instructions for applying the pre-peeling composition prior to applying a peeling composition to the skin. The kits may further comprise a peeling composition.

It is to be understood that both the foregoing general description and the following detailed description are exemplary and explanatory only, and are not restrictive of the invention.

As used herein, the expression "at least one" means one or more and thus includes individual components as well as mixtures/combinations.

"Cosmetically acceptable medium" means a medium that is compatible with any keratin material, such as the skin, the hair, the nails, the eyelashes, the eyebrows, the lips and any other area of body or facial skin.

"Peeling" as used herein includes peeling of skin associated with superficial (light), medium and/or deep chemical peeling procedures. It also includes exfoliation, desquamation and/or sloughing of skin associated with application of keratolytic agents to skin.

The phrase "accelerating the replacement or replenishment of epidermis" for the purposes of the present invention is intended to mean, for example, an increase or enhanced replacement or replenishment of epidermis in the presence of or as caused by the invention peeling compositions and/or peeling methods relative to replacement or replenishment of epidermis in the absence of the peeling compositions. Such acceleration may be of any amount, for example any amount greater than 100% (e.g., 101, 103, 105%) including for example 1000% relative to the replacement or replenishment of epidermis in the absence of the peeling compositions. 100% would denote no acceleration.

The cosmetic compositions and methods of the present invention can comprise, consist of, or consist essentially of the essential elements and limitations of the invention described herein, as well as any additional or optional ingredients, components, or limitations described herein or any otherwise useful ingredient found in personal care compositions intended for application to keratin materials.

The compositions of the present invention may be in any form suitable for use on skin such as, for example, non-solid anhydrous, oil-free or emulsion compositions (for example, water-in-oil emulsion, oil-in-water emulsion, multiple emulsion (W/O/W or O/W/O), nanoemulsions, etc.). The compositions of the present invention can contain colorants such as pigments. Additionally, the compositions of the present invention can be clear or transparent: that is, they can contain little or no colorants. The subject compositions may be liquid, e.g., lotions, semisolid, or solids. Most advantageously they include a gelling agent and are formulated as gels. Preferably, the compositions are designed to be, and capable of being, rinsed off after application.

As defined herein, stability is tested by placing the composition in a controlled environment chamber for 8 weeks at 25°C. In this test, the physical condition of the sample is inspected as it is placed in the chamber. The sample is then inspected again at 24 hours, 3 days, 1 week, 2 weeks, 4 weeks and 8 weeks. At each inspection, the sample is examined for abnormalities in the composition such as phase separation if the composition is in the form of an emulsion. The stability is further tested by repeating the 8-week test at 40°C, 37°C, 45°C, 50°C and/or under freeze-thaw conditions. A composition is considered to lack stability if in any of these tests an abnormality that impedes functioning of the composition is observed. The skilled artisan will readily recognize an abnormality that impedes functioning of a composition based on the intended application.

Pre-peeling composition

According to the present invention, pre-peeling compositions comprising urea and an N-substituted aminosulfonic acid compound are provided.

According to preferred embodiments of the present invention, the pre-peeling composition comprises an N-substituted aminosulfonic acid compound which is preferably an amino derivative of an alkylsulfonic acid. Preferably, the alkyl group has 1 to 10 carbon atoms, more preferably 2 to 6 carbon atoms, and even more preferably from 2 to 3 carbon atoms. Amino derivatives of ethanesulfonic acid and propanesulfonic acid are particularly preferred.

Suitable N-substituted aminosulfonic acid compounds include, but are not limited to, N,N-bis[2-hydroxyethyl]-2-aminoethanesulfonic acid (pKa=7.17), N-2-hydroxyethylpiperazine-N'-2-ethanesulfonic acid (pKa=7.55), 3-[N-morpholino]propanesulfonic acid (pKa=7.15), piperazine-N,N'-bis[2-ethanesulfonic] acid (pKa=6.82), 3-[N-tris(hydroxymethyl)methylamino]-2-hydroxypropanesulfonic acid (pKa=7.6), 2-[N-morpholino]ethanesulfonic acid (pKa=6.15), N-(2-acetamido)-2-aminoethanesulfonic acid (pKa=6.88), N-tris(hydroxymethyl)methyl -2-aminoethanesulfonic acid (pKa=7.5). A mixture of these acids may also be used. Preferably, the N-substituted aminosulfonic acid compound constitutes a buffer with a pKa at approximately 20°C ranging from 6 to 7.8, more preferably from 6.3 to 7.6. These ranges include all specific pKas and subranges therebetween, such as 6.1, 6.2, 6.4, 6.5, 6.8, 7.0, 7.2 and 7.5. The pKa values are determined at approximately 20°C at a concentration of 0.1 M. Hydroxyethylpiperazine ethane sulfonic acid is a particularly preferred N-substituted aminosulfonic acid compound.

Preferably, the N-substituted aminosulfonic acid compound is present in an amount ranging from about 0.1 % to about 50% by weight of the total weight of the composition, more preferably from about 0.5% to about 45% of the total weight of the composition, more preferably from about 1 % to about 40% of the total weight of the composition, and most preferably from about 10% to about 40%, including all ranges and subranges therebetween.

The quantity of urea in the pre-peeling composition may vary within a wide range. Preferably, urea is present in an amount ranging from about 0.1 % to about 50% by weight of the total weight of the composition, more preferably from about 0.5% to about 45% of the total weight of the composition, more preferably from about 1 % to about 40% of the total weight of the composition, and most preferably from about 10% to about 40%, including all ranges and subranges therebetween.

According to preferred embodiments, the pre-peeling composition has a pH ranging from approximately 6.5 to 7.5, more preferably from 6.8 to 7.2, and even more preferably from 6.9 to 7.1. The pH may be adjusted by any suitable means such as, for example, by an inorganic acid or organic acid such as hydrochloric acid, or an inorganic or organic base, such as sodium hydroxide or triethanolamine. Depending on the pH of the pre-peeling composition, the N-substituted aminosulfonic acid compound can be in partial salt form.

According to preferred embodiments of the present invention, the pre-peeling composition further comprises at least one polyol. Any suitable polyol (that is, compound containing more than one hydroxy moiety) can be used in accordance with the present invention.

Examples of suitable polyols include, but are not limited to, glycerol (also known as glycerin); polyalkylene glycols, alkylene polyols and their derivatives (for example, propylene glycol, hexylene glycol, butylene glycol, dipropylene glycol, polypropylene glycol, polyethylene glycol and derivatives thereof) such as, for example, PEG-4, PEG-6, PEG-7, PEG-8, etc.; sorbitol and its derivatives including hydroxypropyl sorbitol; xylitol; 1,2,6-hexanetriol; alkoxylated glycerols such as ethoxylated glycerol and propoxylated glycerol; and mixtures thereof.

Preferably, the amount of polyol (if present) ranges from about 1 % to about 75% by weight of the total weight of the composition, more preferably from about 5% to about 70% of the total weight of the composition, more preferably from about 10% to about 65% of the total weight of the composition, and most preferably from about 15% to about 55%, including all ranges and subranges therebetween.

According to preferred embodiments of the present invention, the pre-peeling composition further comprises water. Preferably, the amount of water (if present) ranges from about 1% to about 75% by weight of the total weight of the composition, more preferably from about 5% to about 70% of the total weight of the composition, more preferably from about 10% to about 65% of the total weight of the composition, and most preferably from about 15% to about 55%, including all ranges and subranges therebetween.

According to particularly preferred embodiments, the pre-peeling composition further comprises both water and polyol.

According to preferred embodiments of the present invention, the pre-peeling composition is substantially free of abrasive compounds (i.e., contains less than about 1 % of abrasive compounds). In another embodiment, the pre-peeling composition is essentially free of abrasive compounds (i.e., contains less than about 0.3% of abrasive compounds). In another embodiment, the pre-peeling composition is free of abrasive compounds (i.e., contains less than about 0.1 % of abrasive compounds). In yet another embodiment, the pre-peeling composition contains no abrasive compounds.

Examples of suitable pre-peeling compositions containing both urea and N-substituted aminosulfonic acid compound as well as a further description of such compositions can be found in U.S. patent 6,303,656, the entire contents of which is hereby incorporated by reference.

Peeling composition

According to the present invention, peeling compositions comprising a peeling agent are provided. Any suitable peeling agent can be used in accordance with the present invention as long as it induces peeling of skin. Advantageously, the peeling agent also accelerates the replacement or replenishment of epidermis.

Preferably, the peeling agent is selected from the group consisting of alpha-hydroxy acid compounds, beta-hydroxy acid compounds, nicotinic acid compounds, and mixtures thereof.

Preferred alpha hydroxy acid compounds include alpha hydroxy acids and related compounds (such as salts and esters). Suitable examples of such compounds are described in the several patents issued to Van Scott and Yu such as, for example, U.S. Pat. No. 5,091,171, which is hereby incorporated by reference in its entirety. The generic structure of alpha-hydroxy acids is as follows:

(Rₐ)(R_{b})C(OH)COOH

wherein Rₐ and R_{b} are H, F, Cl, Br, alkyl, aralkyl or aryl group of saturated or unsaturated, isomeric or non-isomeric, straight or branched chain, having 1 to 25 carbon atoms, or cyclic form having 5 or 6 ring members, and in addition Rₐ and R_{b} may carry OH, CHO, COOH and alkoxy group having 1 to 9 carbon atoms, said alpha hydroxyacid existing as a free acid or lactone form, or in salt form with an organic base or an inorganic alkali, and as stereoisomers, and D, L, and DL forms when Rₐ and R_{b} are not identical.

According to preferred embodiments, the alpha hydroxy acid compound is selected from the group consisting of glycolic acid, lactic acid, malic acid, citric acid, and mixtures thereof. According to particularly preferred embodiments, the alpha hydroxy acid compound is selected from the group consisting of glycolic acid, lactic acid, and mixtures thereof. The most preferred embodiment is where the alpha hydroxy acid compound is glycolic acid.

Preferred beta hydroxy acid compounds include beta hydroxy acids and related compounds (such as salts and esters). Suitable examples of such compounds include beta hydroxy acids of general structure:

(Rₐ)(R_{b})C(OH)CCOOH

wherein Rₐ and R_{b} have the same definition as above in connection with alpha hydroxy acid compounds. A particularly preferred beta hydroxy acid compound is salicylic acid.

Other suitable examples of beta hydroxy acid compounds include, but are not limited to, those described in U.S. patent application publication no. 2004/0161392, the entire contents of which is hereby incorporated by reference. For example, suitable beta hydroxy acid compounds include salicylic acid derivatives of the following formula:

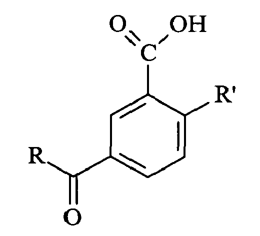

where R is a linear, branched or cyclic saturated aliphatic group or an aliphatic unsaturated group containing one or a number of double bonds, which may or may not be conjugated, these groups containing from 2 to 22, preferably 3 to 11, carbon atoms and being able to be substituted for example by at least one substituent selected from (a) halogen atoms, (b) the trifluoromethyl group, (c) hydroxyl groups in the free form or esterified by an acid having from 1 to 6 carbon atoms or (d) a carboxyl functional group which is free or esterified by a lower alcohol having from 1 to 6 carbon atoms;

R' is a hydroxyl group or an ester functional group of the following formula:

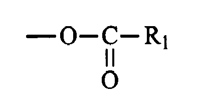

where R₁ is a linear or branched saturated or unsaturated aliphatic group having from 1 to 18 carbon atoms, and salts thereof.

In a preferred embodiment R is a linear, branched or cyclized saturated aliphatic chain containing from 3 to 11 carbon atoms, an unsaturated chain containing from 3 to 17 carbon atoms and containing one or more conjugated or unconjugated double bonds, the abovementioned chains being optionally substituted by one or more halogen atoms or by trifluoromethyl groups, by one or more hydroxyl groups in free form or esterified by an acid containing from 1 to 6 carbon atoms, or by a carboxyl group, free or esterified by a lower alcohol containing from 1 to 6 carbon atoms, these various groups being optionally simultaneously present in the said substituents; and

R' is a hydroxyl group or an ester group of the following formula:

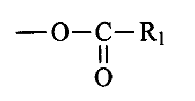

where R₁ is a saturated or unsaturated aliphatic group containing from 1 to 18 carbon atoms.

Preferred salicylic acid derivatives useful herein include those described in U.S. Pat. No. 5,558,871, FR 2,581,542, U.S. Pat. No. 4,767,750, EP 378,936, U.S. Pat. No. 5,267,407, U.S. Pat. No. 5,667,789, U.S. Pat. No. 5,580,549, and EP-A-570,230, all incorporated herein by reference in their entirety. Further, particularly preferred salicylic acid derivatives useful herein include 5-n-octanoyl salicylic acid (capryloyl salicylic acid), 5-n-decanoyl salicylic acid, 5-n-dodecanoyl salicylic acid, 5-n-heptyloxy salicylic acid and 4-n-heptyloxy salicylic acid. A highly preferred salicylic acid derivative is capryloyl salicylic acid (Trade name: Mexoryl SAB); see page 139 of the International Cosmetic Ingredient Dictionary, 6th Edition, Volume 1, published by the Cosmetic Toiletries, and Fragrance Association, 1995, incorporated herein by reference in its entirety.

According to a preferred embodiment, the peeling agent comprises both an alpha hydroxy acid compound and a beta hydroxy acid compound. Examples of suitable peeling agents comprising both an alpha hydroxy acid compound and a beta hydroxy acid compound can be found in U.S. patent application publication no. 2004/0067243, the entire contents of which is hereby incorporated by reference.

Preferred nicotinic acid compounds include nicotinic acid and related compounds (such as salts, amides and esters). Suitable examples of such compounds are described in U.S. patent application publication no. 2005/0152862, the entire contents of which is hereby incorporated by reference. Preferably, the nicotinic acid compounds are used in conjunction with a vitamin C compound as described in U.S. patent application publication no. 2005/0152862.

Preferably, the peeling agent(s) is present in an amount sufficient to effect skin peeling. Typically, such effective amounts range from about 0.5% to about 100% by weight of the total weight of the composition. Those of ordinary skill in the art would recognize that the amount of peeling agent(s) required to effect skin peeling would depend on factors such as the strength of the peeling agent being used. For example, one skilled in the art would recognize that amounts of salicylic acid as low as 0.5% effect skin peeling, and that amounts of glycolic acid as high as 100% effect skin peeling. As would be recognized by those skilled in the art, other factors affecting the ability of a peeling agent to cause skin peeling would include the length of time the peeling agent is left on skin after application (the longer the agent is left on skin, the more peeling occurs, so less peeling agent would be necessary to effect peeling the longer the agent is left on skin). At any rate, preferred ranges of peeling agent concentrations are from about 5% to about 75% of the total weight of the composition, more preferably from about 10% to about 60% of the total weight of the composition, and most preferably from about 20% to about 50%, including all ranges and subranges therebetween. Those skilled in the art will know how to adjust these quantities according to the depth of the peeling which has to be performed, that is to say the layers of epidermis or of dermis which have to be removed, in view of this disclosure.

Additional Ingredients

The pre-peeling and peeling compositions of the present invention can also comprise any additive usually used in the field under consideration. For example, dispersants, antioxidants, essential oils, preserving agents (preservatives) such as, for example phenoxyethanol and paraben derivatives, chelating agents such as EDTA and its derivatives, fragrances, liposoluble polymers that are dispersible in the medium, fillers, neutralizing agents, cosmetic and dermatological active agents, emollients, moisturizers, vitamins, essential fatty acids, sunscreens, film forming agents, colorants, and mixtures thereof can be added. A non-exhaustive listing of such ingredients can be found in U.S. patent application publication no. 2004/0170586, the entire contents of which is hereby incorporated by reference. Further examples of suitable additional components can be found in the other references which have been incorporated by reference in this application, including but not limited to the applications from which this application claims priority. Still further examples of such additional ingredients may be found in the International Cosmetic Ingredient Dictionary and Handbook (9th ed. 2002).

Particularly relevant examples of acceptable additional ingredients for pre-peeling compositions are provided by U.S. patent 6,303,656, while particularly relevant examples of acceptable additional ingredients for peeling compositions are provided by U.S. patent application publication nos. 2004/0161392, 2004/0067243 and 2005/0152862, the entire contents of all of which are hereby incorporated by reference.

A person skilled in the art will take care to select the optional additional additives and/or the amount thereof such that the advantageous properties of the compositions according to the invention are not, or are not substantially, adversely affected by the envisaged addition.

These substances may be selected variously by the person skilled in the art in order to prepare a composition which has the desired properties, for example, consistency or texture.

These additives may be present in the compositions in a proportion from 0% to 99% (such as from 0.01 % to 90%) relative to the total weight of the composition and further such as from 0.1 % to 50% (if present).

Needless to say, the compositions of the invention should be cosmetically or dermatologically acceptable, i.e., it should contain a non-toxic physiologically acceptable medium and should be able to be applied to the skin of human beings. For the purposes of the invention, the expression "cosmetically acceptable" means a composition of pleasant appearance, odor, feel and/or taste.

Preferably, oils, if present, represent from about 5% to about 30% by weight of the total weight of the composition, more preferably from about 10% to about 20% of the total weight of the composition, and most preferably from about 10% to about 15%, including all ranges and subranges therebetween.

Water, when present, preferably represents from about 1 % to about 75% by weight of the total weight of the composition, more preferably from about 5% to about 70% of the total weight of the composition, and most preferably from about 15% to about 55%, including all ranges and subranges therebetween.

According to preferred embodiments, both the peeling composition and the pre-peeling composition further comprise a cosmetically/dermatologically acceptable carrier.

A group of preferred carriers used for the peeling compositions of the invention are dermatologically acceptable liquid solvents in which peeling agents are soluble at high concentrations. In this particular context, the phrase "dermatologically acceptable liquid solvents" has the same definition as set forth above in connection with the pre-peeling composition. Examples of preferred solvents include water, polyols such as alkylene glycols (propylene glycol, polyethylene glycol) and glycerin, and aqueous alcohol. Highly preferred solvents include ethanol and isopropanol. Other useful solvents include methanol, acetone and ether (diethyl ether). Mixtures containing one or more of these solvents or other solvents are included.

According to the present invention, methods for method of peeling skin comprising (a) applying a pre-peeling composition comprising urea and an N-substituted aminosulfonic acid compound to the skin; and (b) subsequently applying a peeling composition to the skin are provided. According to particularly preferred embodiments, the pre-peeling composition is removed from the skin before applying the peeling composition to skin. By "removed from the skin," it is meant that the pre-peeling composition is wiped, washed, rinsed, or cleaned off of skin in some manner. However, it is to be understood that the peeling composition can be applied to skin without first removing the pre-peeling composition from the skin.

According to preferred embodiments, the peeling composition may be applied in any manner, for example, by pasting, spraying, wiping, dispensing, etc. (hereinafter "applying") on the skin to be peeled. This can typically be accomplished with, for example, a spray bottle, an absorbent cotton swab wetted with the composition, with a composition-wetted sable brush or by gentle wiping with a composition-wetted absorbent fibrous material such as a gauze square or nonwoven pad, but other composition application techniques that coat the skin with the composition, preferably in a uniform manner, are also feasible. The application serves as a peel, the degree of which depends upon the amount or concentration of peeling agent and time of application, all of which is within the skill of the ordinary artisan in view of this disclosure. The peeling composition of the invention may in addition be applied not only to the skin to be peeled but also to surrounding areas.

In preferred embodiments, the peeling composition is allowed to air dry over a relatively short period of time, preferably less than 15 minutes and, with the preferred ethanol solvent, typically in the range of about 3 to 10 minutes. Drying may be promoted by directing a gentle stream of air, preferably warm air, onto the treated area or by other analogous procedures. A single uniform application of the composition to the skin to be treated and/or its surroundings is generally sufficient. Additional or multiple applications either before or immediately after the applied composition has dried are normally unnecessary but may be useful in some situations, e.g., in treating skin on other parts of the body other than the face or in treating skin severely in need of peeling.

According to preferred embodiments, once the applied composition has been on the skin for a sufficient amount of time, the peeling composition can be removed from the skin, for example after the composition has dried on the skin, or it may be allowed to remain on the skin for further time, depending on the results desired. When treatment is finished the skin may thereafter be preferably wiped or washed, rinsed, etc., with water etc. to remove any residue or traces of the applied peeling agent or peeling composition, including any deposits of the peeling agent that may remain after drying. This step, however, is not critical but is highly preferred.

In embodiments where a relatively nonvolatile solvent is employed and the peeling composition does not quickly dry on the treated skin, the skin is preferably wiped or washed, rinsed, etc., no later than about one hour, and preferably no more than about 15 minutes, after application of the peeling composition, to remove all traces of the applied solution. This period, of no more than about one hour, and preferably no more than about 15 minutes, is normally more than sufficient to provide the desired benefits resulting from treatment according to this invention, but is not limiting. Time periods can vary widely, as noted above.

Preferably, the treated skin is washed or wiped with water, e.g., with a water-moistened or water-wet swab, gauze square, or the like. Other solutions, such as an aqueous solution of mild detergent, aqueous alcohol solutions or isopropanol or ethanol, and the like, may also be used for this purpose. Additional applications of the peeling composition immediately after the wiping/washing step, followed by drying and repeated wiping/washing, are generally unnecessary, as noted above, but may be desirable in some circumstances.

During the period generally beginning a few days, e.g., about 2 to 5 days, after the invention treatment, a typical patient may experience some peeling and scaling of the treated skin. The peeling and scaling may generally last no more than about 7 days and may be as short as 2 or 3 days in duration. Although the present invention does not require any special steps to be taken during this period, a bland or mild moisturizer may be applied, as desired, to the treated skin to reduce the visibility of scaling, peeling skin and to reduce skin dryness. The skin treated in the method of this invention may be treated further, with conventional skin treatment therapies.

It is to be noted herein that it is possible to apply to the skin the peeling composition after removal of the cuticle, particularly at low amounts/concentrations of peeling agent whereby the cuticle remaining in the hair follicle or in the skin can be removed.

The peeling composition is preferably applied to the skin to be peeled at a temperature of about 15°C to about 30°C, about 20°C to about 25°C being preferred. Depending on carrier/solvent volatility characteristics, a temperature outside of these ranges, e.g., use of lower temperatures for highly volatile materials, may be preferred.

Preferably, the skin to be peeled is first cleaned for example with ethanol, but this step is optional and not essential to the method of this invention. The cleaning may be accomplished by gently wiping the skin with a gauze square wetted with ethanol or acetone for example, before application of the pre-peeling and peeling compositions. This cleaning is intended to degrease the skin and to remove makeup and debris, as well as sebum. Other cleaning or degreasing agents may also be used.

While not bound by theory, it is believed that the invention method can remove the epidermis (mainly the cuticle) of the skin and impose influences upon the cells of the stratum spinosum epidermidis and the stratum basale epidermidis of the epidermis, and cause the reproduction of the fibroblast of the corium. The aged corium portion can be replaced with the reproduced fibroblast. At the same time, the cuticle of the hair follicle can also be peeled off and the accumulated cuticle can be removed. The benefits of chemical peels are further discussed in WO 97/28786, incorporated herein by reference in its entirety.

The treatment time according to this invention is preferably measured as the time of exposure of the treated skin to the peeling composition, with treatment time ending for compositions once the carrier (e.g., volatile solvent) has evaporated from the applied composition or once the still-wet coating of applied composition is wiped or otherwise removed from the skin.

As indicated above, the peeling composition is intended to be used for performing a chemical peeling, preferably superficial, and may be used to attenuate wrinkles and fine lines and/or pigmented spots and/or scars. The peeling composition is preferably used on people with acne and/or wrinkles and/or scars and/or pigmentation defects such as melasmas and senile or actinic lentigo. It may be applied to the face and/or the neck and/or the neck and shoulders and/or the hands and/or the back, or anywhere else on the skin or scalp in need thereof.

According to preferred embodiments, a method of peeling skin comprising the following elements is provided:

(a) topically applying, to an area of human skin, a pre-peeling composition as defined above,

(b) removing the pre-peeling composition,

(c) topically applying, to the same area of human skin, a peeling composition as defined above,

(d) leaving the peeling composition in contact with the skin for a period of between 5 min and 6 hours, and

(e) removing the composition, for example by rinsing.

Other examples of acceptable peeling methods and procedures are provided by U.S. patent application publication nos. 2004/0161392, 2004/0067243 and 2005/0152862, the entire contents of all of which are hereby incorporated by reference.

According to another preferred embodiment of the present invention, methods for preparing skin for peeling comprising applying a pre-peeling composition comprising urea and an N-substituted aminosulfonic acid compound to the skin prior to applying a peeling composition to the skin are provided.

According to another preferred embodiment of the present invention, methods for improving the tactile roughness of skin which has been peeled comprising applying a pre-peeling composition comprising urea and an N-substituted aminosulfonic acid compound to the skin prior to applying a peeling composition to the skin are provided.

According to yet another preferred embodiment of the present invention, methods for minimizing the burning sensation on or in skin which has been peeled comprising applying a pre-peeling composition comprising urea and an N-substituted aminosulfonic acid compound to the skin prior to applying a peeling composition to the skin are provided.

The present invention also envisages kits and/or prepackaged materials suitable for consumer use containing one or more compositions according to the description herein. According to preferred embodiments, a kit comprising: (a) a pre-peeling composition comprising urea and an N-substituted aminosulfonic acid compound; and (b) instructions for applying the pre-peeling composition prior to applying a peeling composition to the skin is provided. The instructions for such a kit could be contained anywhere in the kit such as, for example, on the packaging or on a separate insert within the kit. Such kits may also include other compositions such as, for example, a peeling composition as described above.

The packaging and application device for any subject of the invention may be chosen and manufactured by persons skilled in the art on the basis of their general knowledge, and adapted according to the nature of the composition to be packaged. Indeed, the type of device to be used can be in particular linked to the consistency of the composition, in particular to its viscosity; it can also depend on the nature of the constituents present in the composition, such as the presence of volatile compounds.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective measurements. The following examples are intended to illustrate the invention without limiting the scope as a result. The percentages are given on a weight basis.

All references, patents, applications, tests, standards, documents, publications, brochures, texts, articles, etc. mentioned herein are incorporated herein by reference.

### Example 1 - Pre-peeling composition

| **INGREDIENT** | **%w/w** |
|---|---|
| **Preservatives** | **1.00** |
| **BHT** | **0,10** |
| **Polyols** | **53.00** |
| **UREA** | **10.00** |
| **Chelating agent** | **0,08** |
| **ALCOHOL DENAT.** | **3.00** |
| **BENZOIC ACID** | **0,20** |
| **DIMETHYL SULFONE** | **1.00** |
| **POLYQUATERNIUM-10** | **1.25** |
| **HYDROXYETHYLPIPERAZINE ETHANE SULFONIC ACID** | **10.00** |
| **SODIUM METABISULFITE** | **0,10** |
| **WATER** | **QS to 100%** |
| **Total** | **100.00** |

### Example 2 - Peeling composition

| **INGREDIENT** | **%w/w** |
|---|---|
| **GLYCERIN** | **10.00** |
| **WATER** | **59,75** |
| | |
| **GLYCOLIC ACID** | **30,25** |
| **Total** | **100.00** |

### Example 3 - Evaluation of Peeling Methods

A one-week, single-center, split-face controlled usage study was conducted to evaluate the efficacy and tolerability of a pre-treatment product on Japanese female subjects receiving a 30% glycolic acid peel, as evaluated by clinical grading and subject self-assessment questionnaires.
Eleven female Japanese subjects completed this study. After Baseline clinical grading at Visit 1, subjects had a glycolic acid peel performed by clinic personnel as described below:
- The subject washed her entire face with a cleanser.
- The clinician degreased the subject's entire face with acetone.
- A thin layer of a gentle soothing ointment was applied around the eyes, nose, and lip area.
- The clinician applied the pre-peeling composition of example 1 to the subject's right or left side of the face, as determined by a randomization design. The opposite side of the face is designated as "Untreated."
- The clinician applied 4 to 5 cc of the peeling composition of example 2 (30% Glycolic Acid) to the subject's entire face.
- After waiting approximately 5 minutes, the peeling composition was removed and a neutralizer solution was applied to the subject's entire face.
Subjects used a SPF 20 sunscreen, a moisturizer and a cleanser in place of their regular skin care products for the duration of the study.
Clinic evaluations and forced comparisons were generally conducted at Baseline (prior to glycolic acid peel), approximately 30 to 60 minutes after glycolic acid peel, and at Day 3 and Day 7. Subjects participated in the following procedures at each time point:
- Clinical Grading of Efficacy/Performance Parameters:
   The following parameters were clinically graded on the right and left sides of the face: Mottled Pigmentation, Tactile Roughness (cheeks), Radiance, Skin Laxity, Pore Size, and Overall Facial Appearance. Additionally, at each post-Baseline time point, the clinical grader made a forced comparison, choosing the side of the face with the best general appearance.
   The following parameters were clinically graded on the right and left sides of the face using a 0 to 10 scale (scale anchors are listed in parentheses):
      - Mottled Pigmentation (0 = none, 10 = severe)
      - Tactile Roughness - cheeks (0 = smooth, 10 = rough)
      - Radiance (0 = sallow/dull/matte, 10 = rosy/bright/radiant)
      - Skin Laxity (0 = firm/elastic, 10 = loose)
      - Pore Size (0 = small, 10 = large)
      - Overall Facial Appearance (0 = unhealthy dull skin, 10 = healthy vibrant skin)
   Results showed that a single application of the pre-peeling composition of example 1 prior to application of the peeling composition of example 2 significantly improved tactile roughness and overall appearance of facial skin compared to the use of the peeling composition of example 2 alone. Improvements were observed as early as Day 3 of the study.
      More specifically, comparisons, based on the average change from Baseline, were made between the treated and untreated sides of the face to determine statistically significant (p = 0.05) differences for efficacy/performance and irritation/safety clinical grading parameters. Results of the comparisons showed a significantly greater improvement for the Treated Side of the face for the following parameters at the indicated time points
      - Tactile Roughness: Day 3
      - Overall Appearance: Day 3, Day 7
   Furthermore, forced comparisons showed that the Investigator chose the pre-treated side as appearing better than the untreated side 9 out of 11 times at Visits 2 and 3. More specifically, at the post-acid peel time point and at Day 3 and Day 7, the clinical grader made a forced comparison between the Treated and Untreated sides of the face regarding which side had the best general appearance. Results of the forced comparisons showed that the Treated side was chosen a significantly greater proportion of times than the Untreated side at Day 3 (81.8% v. 18.2%, p-value 0.03) and Day 7 (81.8% v. 18.2%, p-value 0.03). By way of comparison, at visit 1 (post-acid peel) the Untreated side was chosen a greater proportion of time than the Treated side (63.6% v. 36.4%, p-value 0.89).
- Irritation/Safety Parameters:
   Subjects were clinically graded on the right and left sides of the face for objective irritation parameters (erythema, edema, scaling) and subjects assessed subjective irritation parameters (burning, stinging, itching, tightness) on the right and left sides of the face. Results of the irritation grading were recorded using the following scale:
   0 = None
   1 = Mild
   2 = Moderate
   3 = Severe
   At Baseline, after glycolic peel procedures at Visit 1, and on Day 3 and Day 7, subjects were clinically graded on the treated and untreated sides of the face for objective irritation parameters (erythema, edema, scaling) and subjects assessed subjective irritation parameters (burning, stinging, itching, tightness). Results of the clinical grading showed a statistically significant increase in Burning at Day 3 for the Untreated Side of the face.
   - Self-Assessment Questionnaires:
      At Day 3 and Day 7, subjects completed self-assessment questionnaires regarding differences in the appearance of skin on the treated and untreated sides of the face. Results of the questionnaire tabulations showed the following significant differences in subjects' perceptions for differences on the Treated and Untreated sides of the face:
- A significantly greater proportion of subjects responded positively for the Treated Side for the following questions:
   - Treated Side more radiant: Day of Treatment
   - Treated Side smoother: Day 3
   - Treated Side had more even skin tone: Day 3
   - Treated Side has better overall appearance: Day 3, Day 7
- A significantly greater proportion of subjects responded positively for the Untreated Side for the following questions:
   - Treated Side had more visible pores: Day of Treatment
   - Untreated Side had more even skin tone: Day of Treatment
   - Untreated Side had firmer skin: Day of Treatment, Day 3
There were no adverse events reported by subjects during the course of the study. In summary, these results demonstrate:
- A single application of the pre-peeling composition of example 1 prior to application of the peeling composition of example 2 significantly improves tactile roughness and overall appearance of facial skin compared to the use of the peeling composition of example 2 alone. Improvements were observed as early as Day 3 of the study.
- Forced comparisons showed that the Investigator chose the pre-treated side as appearing better than the untreated side 9 out of 11 times at Visits 2 and 3. Forced comparisons made by subjects (using self-assessment questionnaires) gave similar results.

## Claims

1. A cosmetic method of peeling skin comprising (a) applying a pre-peeling composition comprising urea and an N-substituted aminosulfonic acid compound to the skin; and (b) subsequently applying a peeling composition to the skin.

2. The cosmetic method of claim 1, further comprising removing the pre-peeling composition from the skin before applying the peeling composition to skin.

3. The cosmetic method of claim 1 or 2, wherein the N-substituted aminosulfonic acid compound is hydroxyethylpiperazine ethane sulfonic acid.

4. The cosmetic method according to anyone of previous claims, wherein the peeling composition comprises at least one alpha hydroxy acid compound.

5. The cosmetic method of claim 4, wherein the alpha hydroxy acid compound is selected from the group consisting of glycolic acid, lactic acid, malic acid, citric acid, and mixtures thereof.

6. The cosmetic method of claim 5, wherein the alpha hydroxy acid compound is selected from the group consisting of glycolic acid, lactic acid, and mixtures thereof.

7. The cosmetic method of claim 6, wherein the alpha hydroxy acid compound is glycolic acid.

8. The cosmetic method according to anyone of previous claims, wherein the peeling composition comprises at least one beta hydroxy acid compound.

9. The cosmetic method of claim 8, wherein the peeling composition comprises at least one salicylic acid derivative of the formula: 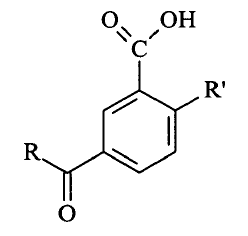 where R is a linear, branched or cyclic saturated aliphatic group or an aliphatic unsaturated group containing one or a number of double bonds, which may or may not be conjugated, these groups containing from 2 to 22 carbon atoms and being able to be substituted by at least one substituent selected from halogen atoms, the trifluoromethyl group, hydroxyl groups in the free form or esterified by an acid having from 1 to 6 carbon atoms or a carboxyl functional group which is free or esterified by a lower alcohol having from 1 to 6 carbon atoms;
R' is a hydroxyl group or an ester functional group of the following formula: 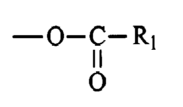 where R₁ is a linear or branched saturated or unsaturated aliphatic group having from 1 to 18 carbon atoms, and salts thereof.

10. The cosmetic method of claim 8, wherein the peeling composition comprises salicylic acid.

11. The cosmetic method according to anyone of previous claims, wherein the peeling composition comprises a nicotine compound.

12. The cosmetic method according to anyone of previous claims, wherein the pre-peeling composition is essentially free of abrasive compounds.

13. The cosmetic method according to anyone of previous claims, wherein the pre-peeling composition comprises at least about 10% to about 40% urea and at least about 10% to about 40% N-substituted aminosulfonic acid compound by weight with respect to the total weight of the composition.

14. The cosmetic method according to anyone of previous claims, wherein the pre-peeling composition further comprises at least one polyol.

15. The cosmetic method of claim 14, wherein the polyol is present in an amount ranging from about 10% to about 60% by weight with respect to the total weight of the composition.

16. A kit comprising (a) a pre-peeling composition comprising urea and N-substituted aminosulfonic acid compound; and (b) instructions for applying the pre-peeling composition prior to applying a peeling composition to the skin.

17. The kit of claim 16, further comprising a peeling composition.

18. The kit of claim 16 or 17, wherein the N-substituted aminosulfonic acid compound is hydroxyethylpiperazine ethane sulfonic acid.

19. The kit according to anyone of claims 16 to 18, wherein the peeling composition comprises at least one peeling agent selected from the group consisting of alpha hydroxy acid compounds, beta hydroxy acid compounds, nicotine compounds, and mixtures thereof.

20. The kit according to anyone of claims 16 to 19, wherein the pre-peeling composition is as defined in anyone of claims 3 to 15..

21. A cosmetic method for preparing skin for peeling comprising applying a pre-peeling composition comprising urea and an N-substituted aminosulfonic acid compound to the skin prior to applying a peeling composition to the skin.

22. An use of at least urea and an N-substituted aminosulfonic acid for preparing a pre-peeling composition to be used prior to applying a peeling composition to a skin.

23. The use of claim 22, wherein the pre-peeling composition is as defined in anyone of claims 3 to 15.

24. The use of claims 22 or 23, wherein the pre-peeling composition is intended to improve the tactile roughness of skin which has been peeled.

25. The use of claims 22 or 23 wherein the pre-peeling composition is intended to minimize the burning sensation on or in skin which has been peeled.
